# EUROPEAN PATENT SPECIFICATION

(11) **EP 0 546 233 B1**
(45) Date of publication and mention of the grant of the patent: **01.03.1995**
(21) Application number: 91810960.4
(22) Date of filing: 10.12.1991
(51) Int. Cl.: C07C 291/10, C07F 13/00, C07B 59/00

(54) **Method for synthesis and 99mTc labelling of 2-alkoxyisobutylisonitrile**
Verfahren zur Herstellung und 99mTc-Markierung von 2-alkoxyisobutylisonitrile
Procédé pour la préparation et marquage avec 99mTc du 2-alkoxyisobutylisonitrile

(43) Date of publication of application: 16.06.1993
(73) Proprietor: INSTITUTE OF NUCLEAR ENERGY RESEARCH, CHINESE ATOMIC ENERGY COUNCIL, Lung-Tan (TW)
(72) Inventor: Te-Wei, Lee, Shih Lin, Taipei (TW); Gann, Ting, Taipei (TW); Chang-Shinn, Su, Tau-Yen (TW); Shyh-Yi, Chyi, Tau-Yen (TW)
(74) Representative: Kiliaridis, Constantin

(56) References cited:
- EP-A- 0 233 368
- US-A- 4 864 051
- APPLIED RADIATION AND ISOTOPES, vol. 42, no. 7, 1991, London, GB; pages 687 - 689; A.J. VAN WYK et al, "Synthesis and 99mTc labelling of MMI(MIBI) and its ethyl analogue EMI"
- "METHODEN DER ORGANISCHEN CHEMIE (HOUBEN WEYL)", 1985 , Thieme Verlag, Stuttgart, DE; pp. 1631-1635

## Description

This invention relates to a novel synthesis of 2-alkoxyisobutylisonitrile, copper isonitrile adducts and radioactive isotope labelling such as ^{99m}Tc.

^{99m}Tc labelling isonitrile compounds have been proven to be myocardial perfusion agents. The synthesis of ether isonitrile ligands has been described by Bergstein et al in European Pat. 233368 issued Oct. 26, 1987. The most useful of ether isonitrile compound is 2-methoxyisobutylisonitrile, synthesis of which is described by Bergstein as follows: 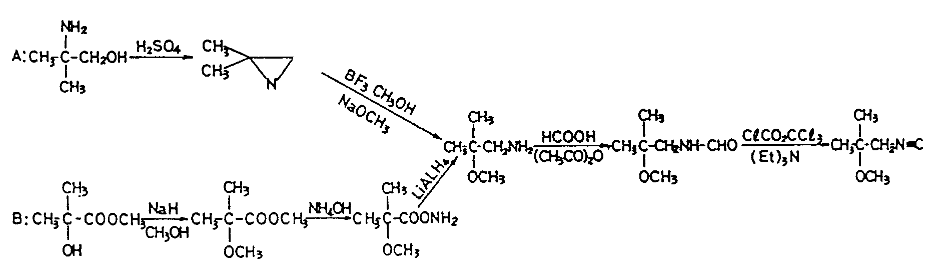
In both of the above (A) and (B) synthetic routes, which include 4 and 5 steps respectively, the total yield is only 5.9 - 8.1%. Van Wyk et al. developed a method to synthesize both 2-methoxyisobutylisonitrile and 2-ethoxyisobutylisonitrile, which was published under the title "Synthesis and ^{99m}Tc Labelling of MMI(MIBI) and its Ethyl Analogue EMI", Appl. Radiat. Isot. 1991, 42:687 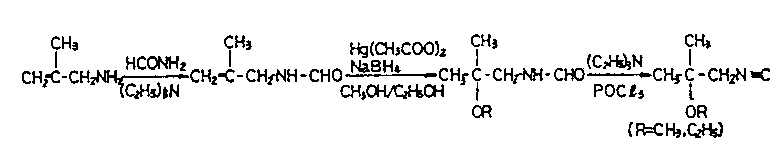
The possible contamination of mercury on the product is a disadvantage of the above process. There is a question of whether or not mercury acetate reagent affects products in the second step. Accordingly, we have invented a new simple and efficient method to synthesize 2-alkoxyisobutylisonitrile compounds.

The main object of the present invention is to provide a new method for synthesis and ^{99m}Tc labelling of 2-alkoxyisobutylisonitrile. Isobutylene is haloalkoxylated with N-halosuccinimide in the presence of alcohol solution to give 2-alkoxyisobutylhalide. This is followed by reaction of potassium phthalimide with 2-alkoxyisobutylhalide, following by hydrazinolysis with hydrazine to yield 2-alkoxyisobutylamine. Finally, in the basic condition, the reaction of 2-alkoxyisobutylamine with chloroform in the presence of catalyst benzyltriethylammonium chloride produces 2-alkoxyisobutylisonitrile.

The new synthesis method of these isonitriles has also proved to be more clean, more efficient and convenient than known methods in the literature.

The ^{99m}Tc labelling of 2-alkoxyisobutylisonitrile is performed by mixing copper isonitrile adducts with radioactive isotope ^{99m}Tc. Such an adduct is labelled easily and rapidly with ^{99m}Tc and produces good yields. Tetrakis(2-alkoxyisobutylisonitrile)copper(I)tetrafluoroborates can be prepared by the exchange of acetonitrile molecules in tetrakis(acetonitrile) copper(I)tetrafluoroborate with 2-alkoxyisobutylisonitrile ligand at room temperature.

The haloalkoxylation of isobutylene can be achieved by halogen in alcohol as shown in equation (1). 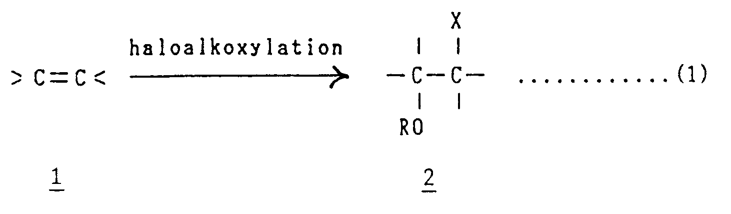
This procedure provides a convenient and high yields 2 from the olefin. Reaction of potassium phthalimide with 2 leads to the N-alkylphthalimide. N-Substituted phthalimides may be converted into the corresponding 3 by hydrolysis or hydrazinolysis. Synthesis of 3 may be summarized schematically as equation (2). 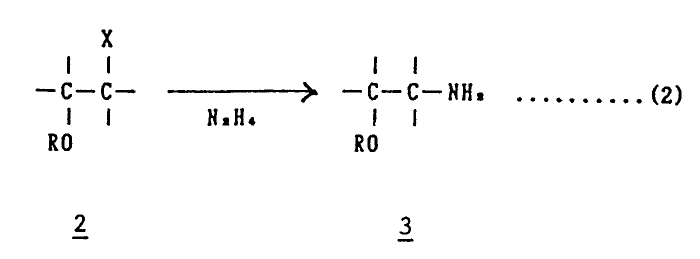
It is obvious that 3 formed in this reaction will be uncontaminated by secondary or tertiary amines.
The phase-transfer catalysis method has been utilized effectively for synthesis of isonitriles. 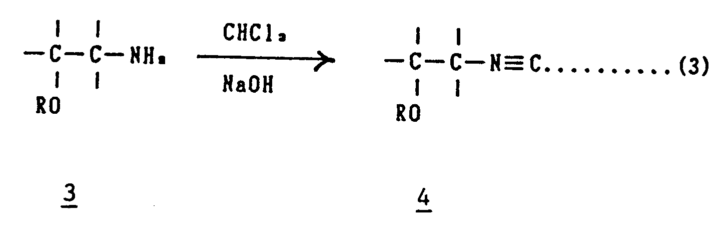
The reaction between 3 and chloroform in NaOH solution and catalyst benzyltriethylammonium chloride gives 4 as shown in equation (3). This invention can be further described by the following examples in which the percentages are expressed by weight unless otherwise indicated.

### Example 1

### Synthesis of 2-methoxyisobutylbromide:

N-Bromosuccinimide (3.56g, 0.02 mol) was dissolved in methanol. The solution was cooled to -10°C in an ice/acetone bath. Isobutylene was slowly introduced and stirred for 5 hours and poured into separatory funnel containing saturated NaCl water. The organic layer was removed and aqueous layer was extracted with three 100 ml portions of dichloromethane. The combined organic extractants was dried over anhydrous magnesium sulfate and filtered, and the solvent was mostly removed by rotary evaporatory. The resulting solution was distilled at atmospheric pressure and the product collected at 140°C (3.14g, 94% yield).
- IR(neat/ν cm⁻¹):: 2960, 2920, 2820, 1450, 1415, 1370, 1360, 1090, 1065, 735, 660
- ¹HNMR(200MHz, CDC1₃/δ ppm):: 1.30(s,6H,2CH₃), 3.24(s, 3H, OCH₃), 3.41(s, 2H, CH₂)
- ¹³CNMR(200MHz, CDC1₃/δ ppm):: 23.71(s, 2CH₃), 40.91(s, CH₂), 49.79(s, OCH₃), 73.63(s,

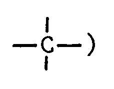

### Example 2

### Synthesis of 2-methoxyisobutyliodide:

N-Iodosuccinimide(4.50g, 0.02 mol) was dissolved in methanol. This was cooled to -15°C in an ice/acetone bath. Isobutylene was slowly introduced and stirred for 4 hours and poured into separatory funnel containing saturated NaCl water. The organic layer was removed and aqueous layer was extracted with three 100 ml portions of dichloromethane. The combined organic extractants was dried over anhydrous magnesium sulfate and filtered, and the solvent was mostly removed by rotary evaporatory. The resulting solution was concentrated under reduced pressure at 10mm Hg pressure and 50°C temperature(4.07g, 95% yield).
- IR(neat/ν cm⁻¹):: 2980, 2950, 2840, 1470, 1420, 1380, 1365, 1095, 1070, 740, 620.
- ¹HNMR(200MHz, CDCl₃/δ ppm):: 1.26(s,6H,2CH₃), 3.15(s, 3H, OCH₃) 3.24(s, 2H, CH₂)

### Example 3

### Synthesis of 2-methoxyisobutylamine:

Potassium phthalimide(3.70g, 0.02 mol) was added to a solution of 2-methoxyisobutylbromide(3.34g, 0.02 mol) in 100 ml of dimethylformamide. Stirring and reflux were continued for 4 hours, and the temperature dropped slowly to 25°C. After the addition of 200 ml of chloroform, the mixture was poured into 500 ml of cold water. The aqueous phase was separated and extracted with two 50 ml portions of chloroform. The combined chloroform extractants were washed with 100 ml of 0.2 N sodium hydroxide and 100 ml of water. After drying the chloroform was removed. The residue was added to hydrazine (2g, 0.04 mol) in 100 ml of methanol and was heated under reflux for an hour. The methanol was removed by concentration under reduced pressure. Concentrated hydrochloric acid was added to the residual aqueous solution and the mixture was heated under reflux for an hour. The solution was then concentrated under reduced pressure to remove most of the hydrochloric acid. The moist residue was adjusted pH=14 using sodium hydroxide and was poured into separatory funnel containing saturated K₂CO₃ solution. The resulting solution was distilled at atmospheric pressure and the product collected at 125°C (1.6g, 78% yield).
- IR(neat/ν cm⁻¹):: 3280, 3065, 2960, 2920, 1640, 1430, 1365, 1075.
- ¹NHMR(200MHz, CDCl₃/δ ppm):: 1.13(s,6H,2CH₃), 1.17(s, 2H, NH₂), 2.61(s, 2H, CH₂), 3.2(s, 3H, OCH₃).
- ¹³CNMR(200MHz, CDCl₃/δ ppm):: 22.54(s, 2CH₃), 49.39(s, CH₂), 50.50(s, OCH₃), 74.98(s,

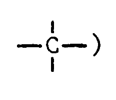

### Example 4

### Synthesis of 2-methoxyisobutylisonitrile:

A mixture of 2-methoxyisobutylamine(2.06g,0.02 mol), chloroform (4,80g, 0.04 mol) benzyltriethylammonium chloride (40mg,0.17 mmol) in 50 ml of dichloromethane, was added dropwise into a flask containing sodium hydroxide solution (3.2g NaOH and 5ml H₂O). The mixture solution was heated under reflux for two hours. After the reaction mixture was diluted with 100 ml of ice water, the organic layer was separated and retained, and the aqueous layer was extracted with 50 ml of dichloromethane. The dichloromethane solutions were combined and dried over anhydrous magnesium sulfate. The resulting solution was concentrated under reduced pressure at 25mm Hg pressure and the product collected at 55°- 60°C(1.42g, 63% yield).
- IR(neat/ν cm⁻¹):: 2980, 2940, 2830, 2150, 1460, 1435, 1390, 1370, 1080.
- ¹HNMR(200MHz, CDCl₃/δ ppm):: 1.28(s,6H,2CH₃), 3.26(s, 3H, OCH₃), 3.38(t, 2H, CH₂)
- ¹³CNMR(200MHz, CDCl₃/δ ppm):: 22.47(s, 2CH₃), 49.97(s, OCH₃), 50.57(t, CH₂), 73.34(s,

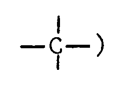

### Example 5

### Synthesis of 2-ethoxyisobutylbromide:

N-Bromosuccinimide (3.56g, 0.02 mol) was dissolved in ethanol. The product was obtained by procedures analogous to those described in Example 1(95% yield).
- IR(neat/ν cm⁻¹):: 2980, 2940, 2900, 2880, 1460, 1440, 1380, 1360, 1120, 1068.
- ¹HNMR(200MHz, CDCl₃/δ ppm):: 1.19(t,3H,CH₃), 1.31(s,6H,2CH₃), 3.44(m, 4H, CH₂ and OCH₂)
- ¹³CNMR(200MHz, CDCl₃/δ ppm):: 16.01(s, CH₃), 24.48(s,2CH₃), 41.23(s,CH₂Br),57.31(S,OCH₂), 73.52(s,

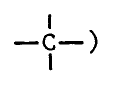

### Example 6

### Synthesis of 2-ethoxyisobutylamine:

The product was obtained by procedures analogous to those described in Example 3(75% yield).
- IR(neat/ν cm⁻¹):: 3380, 2980, 2950, 2850, 1660, 1470, 1430, 1398, 1370, 1120, 1075.
- ¹HNMR(200MHz, CDCl₃/δ ppm):: 1.15(m, 11H, 2CH₃, CH₃ and NH₂), 2.60(s, 2H, CH₂), 3.38(q,2H,OCH₂)
- ¹³CNMR(200MHz, CDCl₃/δ ppm):: 16.26(s,CH₃), 23.25(s, 2CH₃), 50.92(s,CH₂), 56.71(s, OCH₃), 73,79(s,

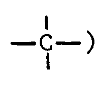

### Example 7

### Synthesis of 2-ethoxyisobutylisonitrile:

The product was obtained by procedures analogous to those described in Example 4 (60% yield).
- IR(neat/ν cm⁻¹):: 2980, 2930, 2900, 2870, 2150, 1475, 1450, 1385, 1360, 1120, 1070.
- ¹HNMR(200MHz, CDCl₃/δ ppm):: 1.14(t, 3H, CH₃), 1.28(s, 6H, 2CH₃), 3.37(t, 2H, CH₂), 3.45(q, 2H, OCH₂)
- ¹³CNMR(200MHz, CDCl₃/δ ppm):: 15.99(s, CH₃), 23.22(s, 2CH₃), 50.89(t, CH₂), 57.57(s, OCH₃), 73.14(s,

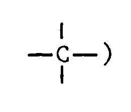

### Example 8

### Synthesis of 2-propoxyisobutylbromide:

N-Bromosuccinimide (3.56, 0.02 mol) was dissolved in 1-propanol. The product was obtained by procedures analogous to those described in Example 1 (93% yield).
- IR(neat/ν cm⁻¹):: 2980, 2945, 2880, 1465, 1430, 1380, 1370, 1100, 1080, 675.
- ¹HNMR(200MHz, CDCl₃/δ ppm):: 0.91(t, 3H, CH₃), 1.29(s,6H,2CH₃), 1.54(m, 2H, CH₂), 3.27(t,2H,OCH₂), 3.39(s, 2H, CH₂Br).
- ¹³CNMR(200MHz, CDCl₃/δ ppm):: 10.84(s,CH₃), 23.72(s,CH₂), 24.52(s,2CH₃), 41.47(s,CH₂Br), 63.76(s,OCH₂), 73.48(s,

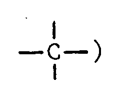

### Example 9

### Synthesis of 2-isopropoxyisobutylbromide:

N-Bromosuccinimide(3.56g, 0.02 mol) was dissolved in 2-propanol. The product was obtained by procedures analogous to those described in Example 1 (93% yield).
- IR(neat/ν cm⁻¹):: 2980, 2955, 2880, 1470, 1430, 1380, 1370, 1120, 670.
- ¹HNMR(200MHz, CDCl₃/δ ppm):: 1.10(d, 6H, 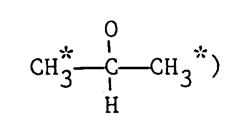 1.30(s, 6H, 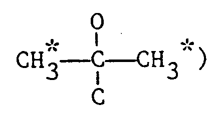 3.35(s, 2H, CH₂), 3.79(m, 1H, CH)
- ¹³CNMR(200MHz, CDCl₃/δ ppm):: 25.10 and 25.17(s,CH₃), 42.21(s,CH₂), 64.65(s,OCH), 74.55(s,

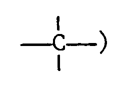

### Example 10

### Synthesis of 2-propoxyisobutylisonitrile:

The product was obtained by procedures analogous to those described in Example 4 (55% yield).
- IR(neat/ν cm⁻¹):: 2980, 2950, 2880, 2160, 1470, 1380, 1370, 1120, 1080.
- ¹HNMR(200MHz, CDCl₃/δ ppm):: 0.92(t,3H,CH₃), 1.28(s,6H,2CH₃), 1.57(m,2H,CH₂), 3.28(m,2H,OCH₂), 3.36(t,2H,CH₂-N≡C)
- ¹³CNMR(200MHz, CDCl₃/δ ppm):: 10.79(s,CH₃), 23.21(s,2CH₃), 23.64(s,CH₂), 50.97(t,CH₂-N), 63.86(s,OCH₂), 73.01(s,

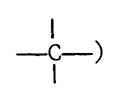

### Example 11

### Preparation of tetrakis(2-methoxyisobutylisonitrile) copper(I) tetrafluoroborate:

Tetrakis(acetonitrile)copper(I)tetrafluoroborate(0.50g, 1.6x10⁻³mol) was suspended in 100 ml of ethanol. 2-Methoxyisobutylisonitrile (0.72g, 6.4x10⁻³mol) was slowly added and stirred at room temperature for an hour. The solvent was then evaporated completely under reduced pressure. The product was recrystallized from ethanol/ether (0.91g, 95% yield), M.P. 100 - 101°C. Anal. Calcd. for C₂₄H₄₄N₄O₄CuBF₄:C,47.80;H,7.30; N,9.29; Cu,10.54; B,1.79; F,12.62. Found: C,47.69; H,7.40; N,9.05; Cu,10.52; B,1.80; F,12.71.
- IR(Nujol mull/ν cm⁻¹):: 2200
- ¹HNMR(200MHz, CDCl₃/δ ppm):: 1.29(s,6H,2CH₃), 3.25(s,3H,OCH₃), 3.61(s,2H,CH₂)
- ¹³CNMR(200MHz,CDCl₃/δ ppm):: 22.60(s,2CH₃), 50.09(s,OCH₃), 51.72(d,CH₂), 73.42(s,

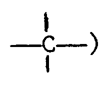

### Example 12

### Preparation of tetrakis(2-ethoxyisobutylisonitrile)copper(I) tetrafluoroborate:

To a stirred suspension of tetrakis(acetonitrile)copper(I) tetrafluoroborate(0.50g, 1.6x10⁻³mol) in 100 ml of ethanol at room temperature, was slowly added 2-ethoxyisobutylisonitrile (0.81g, 6.4x10⁻³mol). After the reaction mixture was stirred for 30 minutes to give a clear solution, the solvent was then evaporated to dryness under reduced pressure. The product was recrystallized from ethanol/n-hexane, and the white solids obtained were washed with n-hexane and dried in vacuo; (1.01g, 96% yield),M.P. 76°- 77°C Anal. Calcd for C₂₈H₅₂N₄O₄CuBF₄: C,51.04; H,7.90; N,8.51; Cu,9.65; B,1.64; F,11.54. Found: C,51.10; H,7.85; N,8.59; Cu,9.61; B,1.70; F,11.61.
- IR(Nujol mull/ν cm⁻¹):: 2200
- ¹HNMR(200MHz, CDCl₃/δ ppm):: 1.18(t,3H,CH₃), 1.29(s,6H,2CH₃), 3.43(q,2H,OCH₂),3.59(s,2H,CH₂)
- ¹³CNMR(200MHz, CDCl₃/δ ppm):: 16.06(s,CH₃), 23.38(s,2CH₃), 52.03(s,CH₂), 57.66(s,OCH₂), 73.18(s,

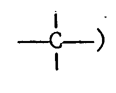

### Example 13

### Preparation of Tc-99m-alkoxyisobutylisonitrile complexes:

In a 8 ml-vial are mixed tetrakis(2-methoxyisobutylisonitrile) copper(I)tetrafluoroborate(1-2mg) or tetrakis(2-ethoxyisobutylisonitrile)copper(I)tetrafluoroborate (1-2mg), sodium citrate dihydrate(2.2-3.2mg), mannitol(16-26mg), cysteine hydrochloride(1-3mg) and stannous chloride(0.05-0.09mg). The vials were sealed and 25-40 mCi(1-2ml) ^{99m}TcO₄⁻obtained by elution of a ⁹⁹Mo/^{99m}Tc radionuclide generator was added. The vials were heated in a 95°- 100°C water bath for 10-15 min. and allowed to cool to room temperature. Quality assurance of in vitro stability was done on ITLC(SG) with saline and methylethylketone(MEK) to determine ^{99m}TcO₂(Rf:0), ^{99m}TcO₄⁻(Rf:0.9 - 1.0) and the ^{99m}Tc isonitrile complex (MEK:Rf:MMI 0.45, EMI 0.8; saline:0).

## Claims

1. A method for synthesis of 2-alkoxyisobutylisonitrile using isobutylene as the starting material, characterized in that haloalkoxylation of said isobutylene gives 2-alkoxyisobutylhalide which is then converted to 2-alkoxyisobutylamine, said 2-alkoxyisobutylamine in the basic condition, on reaction with chloroform producing 2-alkoxyisobutylisonitrile in which the product of 2-alkoxyisobutylisonitrile has a general formula 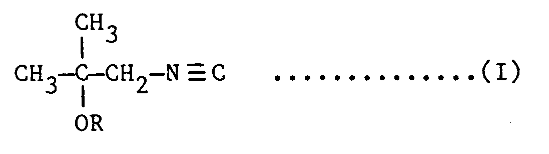 wherein
R is an alkyl group having 1-4 carbon atoms.

2. The method of claim 1 wherein said 2-alkoxyisobutylhalide is synthesized by reacting isobutylene with N-halosuccinimide at temperature of -50°- 0°C in the corresponding alcohol solvent.

3. The method of claim 1 wherein said 2-alkoxyisobutylamine is synthesized by reacting 2-alkoxyisobutylhalide with potassium phthalimide and hydrazine at temperature of 100°- 200°C.

4. The method of claim 1 wherein said 2-alkoxyisobutylisonitrile is synthesized by reacting 2-alkoxyisobutylamine with chloroform, sodium hydroxide and catalyst benzyltriethylammonium chloride at 50°- 100°C temperature.

5. The method of claim 1 wherein said structure (I) with copper salt give a complex which is labelled with technetium-99m in the presence of Sn(II) and the labelled yield is higher than 90%.

## Patentansprüche

1. Synthesemethode von 2-Alkoxyisobutylisonitril mit Isobutylen als Ausgangsmaterial, dadurch gekennzeichnet, dass durch Haloalkoxylation des genannten Isobutylens 2-Alkoxyisobutylhalid erhalten wird, welches dann zu 2-Alkoxyisobutylamin versetzt wird, wobei das genannte 2- Alkoxyisobutylamin, unter basischen Bedingungen, mit Chloroform reagiert und 2-Alkoxyisobutylisonitril ergibt, wobei das genannte 2-Alkoxyisobutylisonitril-Produkt die allgemeine Formel 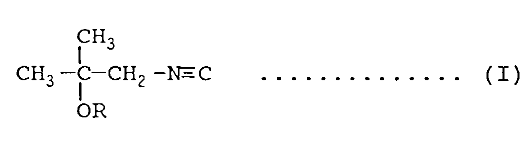 aufweist, wobei R eine Alkylgruppe mit 1 bis 4 Kohlenstoffatomen darstellt.

2. Synthesemethode gemäss Anspruch 1, in der das genannte 2-Alkoxyisobutylhalid durch Reaktion von Isobutylen mit N-Halosuccinimid, bei Temperaturen zwischen -50°C bis 0°C, in einem entsprechenden alkoholischen Lösungsmittel, synthetisiert wird.

3. Synthesemethode gemäss Anspruch 1, bei der das genannte 2-Alkoxyisobutylamin durch Reaktion von 2-Alkoxyisobutylhalid mit Kaliumphthalimid und Hydrazin bei einer Temperatur zwischen 100°C bis 200°C synthetisiert wird.

4. Synthesemethode gemäss Anspruch 1, in der das genannte 2-Alkoxyisobutylisonitril durch Reaktion von 2-Alkoxyisobutylamin mit Chloroform, Natriumhydroxid und dem Katalisator Benzyltriethylammoniumchlorid bei einer Temperatur von 50°C bis 100°C synthetisiert wird.

5. Synthesemethode gemäss Anspruch 1, in der die genannte Struktur 1 mit einem Kupfersalz einen Komplex ergibt, welches mit Technetium-99m in Anwesenheit von Sn(II) markiert wird und die Markierungsausbeute grösser ist als 90%.

## Revendications

1. Méthode de synthèse de 2-alkoxyisobutylisonitrile utilisant l'isobutylène comme produit de départ, caractérisée en ce que par haloalkoxylation dudit isobutylène on obtient un 2-alkoxyisobutylhalide qui est ensuite converti en 2-alkoxyisobutylamine, que ladite 2-alkoxyisobutylamine, en conditions basiques, en réagissant avec du chloroforme produit un 2-alkoxyisobutylisonitrile, le produit 2-alkoxyisobutylisonitrile ayant la formule générale 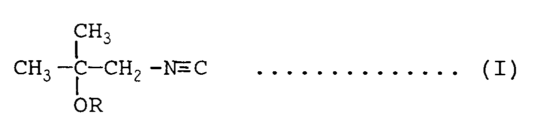 dans laquelle R désigne un groupe alkyle ayant de 1 à 4 atomes de carbone.

2. Méthode selon la revendication 1, dans laquelle ledit 2-alkoxyisobutylhalide est synthétisé en faisant réagir l'isobutylène avec un N-halosuccinimide à une température comprise entre -50° C et 0° C dans le solvant alcoolique correspondant.

3. Méthode selon la revendication 1, dans laquelle ladite 2-alkoxyisobutylamine est synthétisée en faisant réagir un 2-alkoxyisobutylhalide avec du phthalimide de potassium et de l'hydrazine à une température entre 100° et 200° C.

4. Méthode selon la revendication 1, dans laquelle le 2-alkoxyisobutylisonitrile est synthétisé en faisant réagir la 2-alkoxyisobutylamine avec du chloroforme, de l'hydroxide de sodium et, comme catalyseur, du chlorure de benzyltriethylammonium à une température de 50° à 100° C.

5. Méthode selon la revendication 1, dans laquelle ladite structure (I) donne, avec un sel de cuivre, un complexe qui est marqué avec du technetium-99m en présence de Sn(II), le rendement de produit marqué étant supérieur à 90%.
